# EUROPÄISCHE PATENTSCHRIFT

(11) **EP 1 212 402 B1**
(45) Veröffentlichungstag und Bekanntmachung des Hinweises auf die Patenterteilung: **02.03.2005**
(21) Anmeldenummer: 00960656.7
(22) Anmeldetag: 13.09.2000
(51) Int. Cl.: C12M 1/04, B09B 3/00

(54) **BIOREAKTOR ZUR FERMENTIERUNG VON FESTEN STOFFEN**
BIOREACTOR FOR FERMENTING SOLIDS
BIOREACTEUR DE FERMENTATION DE MATIERES SOLIDES

(30) Priorität: 13.09.1999 DE 19943853
(43) Veröffentlichungstag der Anmeldung: 12.06.2002
(73) Patentinhaber: Höfer Bioreact GmbH, 53115 Bonn (DE)
(72) Erfinder: Hölker, Udo, 53639 Königswinter-Rauschendorf (DE)
(74) Vertreter: Helbing, Jörg, Dr. Dipl.-Chem.
(86) Internationale Anmeldenummer: PCT/EP2000/008929
(87) Internationale Veröffentlichungsnummer: WO 2001/019954

(56) Entgegenhaltungen:
- AT-B- 395 686
- AT-B- 395 859
- US-A- 5 153 137

## Beschreibung

Die vorliegende Erfindung betrifft einen Bioreaktor zur Fermentierung fester Stoffe sowie ein entsprechendes Fermentationsverfahren.

Der Umsatz von festen, wasserunlöslichen oder partikulären Substraten in Fermentern birgt eine Vielzahl an Problemen, welche in erster Linie die Belüftung, die Durchmischung und die Nährmedienzugabe betreffen. Wenn der umzusetzende Stoff von verschiedenen Mikroorganismen synergistisch angegriffen werden soll, ist zudem eine gezielte Versorgung des Reaktionsraums mit Nährstoffen und Sauerstoff erforderlich. In großvolumigen Reaktoren lassen sich diese Fermentationen derzeit aufgrund der erschwerten Durchmischung und der damit verbundenen mangelhaften Belüftung und Unterversorgung mit Substraten, nicht realisieren.

Die Durchmischung findet in handelsüblichen Reaktorsystemen durch mechanische Rührsysteme statt. Darüber hinaus beschreibt das US-Patent 4,846,964 ein Fließbett-Bioreaktorsystem zur Umwandlung von Kohle in mikrobiologisch verflüssigte Kohleprodukte, bei dem ein aufstrebender wäßriger Strom die Kohlepartikel in Lösung hält. Die vorstehenden Mischmethoden haben den Nachteil, dass bei einer Fermentation von höheren Substratkonzentrationen bzw. Substraten, die zur Verklumpung neigen, eine hinreichende Durchmischung und somit ein hoher Substratumsatz nicht mehr gegeben ist.

Aus dem US-Patent 5,153,127 ist darüber hinaus ein Feststoffreaktor zur Behandlung von Kompost beschrieben, bei dem mittels lanzenförmiger Düsen das Substrat belüftet wird. AT-B-395 680 und AT-B-395-859 beschreiben ein Verfahren zur Durchlüftung von Mülldeponien, wobei sauerstoffhaltiges Gas/Luft in das deponiegelagerte Material eingeleitet/eingeblasen wird.

Die Aufgabe der vorliegenden Erfindung bestand nun darin, einen Bioreaktor und ein Fermentationsverfahren zur Verfügung zu stellen, die die Nachteile des Standes der Technik nicht mehr aufweisen, insbesondere die eine hinreichende Durchmischung des zu fermentierenden Materials gewährleisten.

Überraschenderweise wurde nun gefunden, dass in einem Bioreaktor durch gezieltes Einleiten von Druckgas, kontinuierlich oder durch Druckgaspulse, eine hinreichende Durchmischung eines Reaktionsmediums, das feste bzw. wasserunlösliche Fermentationsstoffe enthält, gegeben ist.

Zur Lösung der gestellten Aufgabe dienen ein Bioreaktor mit den Merkmalen des Anspruchs 1 sowie ein Verfahren zur aeroben Fermentierung mit den Merkmalen des Anspruchs 19.

Der Bioreaktor dient zur Fermentierung wasserunlöslicher oder partikulärer Substrate wie z. B. Holz (welches aufgrund seines Ligninanteils nur von bestimmten Mikroorganismen abbaubar ist), Kohlen (mit dem Ziel, die flüssigen Fermentationsprodukte als Ausgangsstoffe für die chemische Industrie oder zur thermischen Nutzung zu verwenden), zur Sanierung von mit Xenobiotika belasteten Böden, zur schnellen Fermentierung von organischen Abfallprodukten, zur biologischen Abwasserreinigung und zur Vorbehandlung von Grundstoffen der chemischen Industrie.

Der vorgeschlagene Bioreaktor erlaubt durch mindestens eine spezielle Düsenanordnung, welche in das zu fermentierende Substrat eingebracht wird und pneumatisch mit Druckgas versorgt wird, erstmals die für Mikroorganismen nötige optimale Belüftung und Durchmischung von festen, wasserunlöslichen oder partikulären Substraten. Sowohl die Versorgung der Mikroorganismen mit Luftsauerstoff als auch deren Versorgung mit Nährmedien, Co-Substraten, Vitaminen, Mineralstoffen, Puffern oder Antibiotika verläuft über ein einzelnes pneumatisches Drucksystem. Der Bioreaktor kann in leicht modifizierter Form in jeder beliebigen Größe vom fünf Liter Labormaßstab bis hin zum großindustriellen mehrstelligen Hektoliterbereich genutzt werden. Der vorgeschlagene Bioreaktor kann mit einfachen Veränderungen ebenfalls als konventioneller Flüssig-/Festphasen-, Festphasen-, Rieselfilm-, "fedbatch" oder Airliftreaktor genutzt werden.

Erfindungsgemäß ist eine erste, sich vertikal erstreckende Düsenanordnung in den Reaktionsraum des Fermentationsbehälters ein- und ausfahrbar. Die Düsenanordnung ist somit in vertikaler Richtung verschiebbar, sodass es auch möglich ist, die Düsenanordnung, während sie mit Druckgas beaufschlagt ist, zur besseren Durchmischung und Belüftung zu bewegen. Falls der Fermentationsvorgang jedoch unter sterilen Bedingungen ablaufen soll, ist es unumgänglich, dass der Reaktionsraum geschlossen bleibt. Die Düsenanordnung besteht aus vertikal in den Fermentationsbehälter hineinragenden Leitungsrohren, die an ihren unteren Enden mit Düsen versehen sind. Auf diese Weise lässt sich das Druckgas bzw. der flüssige bioreaktive Stoff in Bodennähe des Fermentationsbehälters einbringen. Die Düsenanordnung ist auch zum Penetrieren von in der Fermentationslösung befindlichen körnigen Feststoffen geeignet. Die vertikalen Leitungsrohre können eine unterschiedliche Länge aufweisen und sind auswechselbar. Ferner kann die Düsenanordnung, wenn sie aus dem Fermentationsbehälter herausgezogen ist auf einfache Weise gereinigt werden.

Zusätzlich ist eine zweite horizontale Düsenanordnung vorgesehen sein, die aus sich horizontal und zueinander parallel durch den Reaktionsraum erstreckenden, miteinander verbundenen Leitungsrohren besteht. Die Leitungsrohre haben auf ihrer Mantelfläche verteilt angeordnete Düsen.

Die horizontale Düsenanordnung kann zusätzlich zu der vertikalen Düsenanordnung zum Durchmischen verwendet werden.

Falls die horizontale Düsenanordnung zum besseren Durchmischen des Fermentationsstoffes rotieren soll, muss die vertikale Düsenanordnung nach oben gefahren werden oder die vertikalen Leitungsrohre müssen so gewählt werden, dass sie ein Rotieren der horizontalen Düsenanordnung nicht behindern, d. h., die "normalen" Leitungsrohre müssen durch kürzere Leitungsrohre ersetzt werden.

Eine vorteilhafte Weiterbildung des erfindungsgemäßen Bioreaktors weist eine Messvorrichtung auf, in der in einer Messkammer beispielsweise mehrere Messelektroden zum Messen eines aus dem Fermentationsbehälter entnommenen Mediums vorgesehen sind. Die Messkammer ist über eine Zufuhrleitung mit dem Fermentationsbehälter, zum Zuführen von zu messendem Medium aus dem Fermentationsbehälter in die Messkammer, verbunden. Um das Medium nach dem Messen wieder in den Fermentationsbehälter zurückzuführen ist die Messkammer ferner über eine Rückführleitung mit dem Fermentationsbehälter verbunden. Das Besondere dieser Anordnung ist, dass der Messkammer eine Druckkammer vorgeschaltet ist, in der sich ein definierbarer Druck aufbauen kann, der dem verdrängten Volumen in der Messkammer entspricht. Das Rückführen des gemessenen Mediums erfolgt erfindungsgemäß dadurch, dass die Messkammer mit Druck beaufschlagt wird, so dass das Medium in den Fermentationsbehälter zurückgedrückt wird.

Bei bekannten Bioreaktoren erfolgen Messungen üblicherweise direkt in dem Fermentationsbehälter. Da in dem Fermentationsbehälter feste Substrate enthalten sind, werden die Messelektroden häufig beschädigt. Bei einer externen Messung wird das zu messende Medium bei bekannten Bioreaktoren durch peristaltische Pumpen in eine Messkammer gefördert. Derartige Pumpen unterliegen hohem Verschleiß und sind zur Förderung größerer Volumina nicht geeignet.

Durch das erfindungsgemäße Fördern des Mediums mit Druck können derartige verschleißbehaftete und damit wartungsintensive Pumpen entfallen. Ferner ist es möglich große Volumina zu fördern. Ein weiterer Vorteil der Förderung mittels Druck ist, dass die Sterilität nicht beeinflusst wird. Dies bedeutet, dass beim Pumpvorgang keine Fremdstoffe in den Fermentationsbehälter gelangen.

Die Zufuhr von Medium über die Zuführleitung in die Messkammer kann durch Unterdruck erfolgen. Die erfindungsgemäße Messkammer kann ferner als zusätzliches Umwälzsystem genutzt werden. Mit einem derartigen Umwälzsystem kann eine zusätzliche Belüftung der in dem Fermentationsbehälter befindlichen Substrate erfolgen.

Weitere vorteilhafte Ausbildungen des Bioreaktors sind den weiteren Unteransprüchen zu entnehmen.

In dem erfindungsgemäßen Verfahren wird zur aeroben Fermentierung fester Stoffe ein diese feste Stoffe enthaltendes Reaktionsmedium durch von oben in das Reaktionsmedium zugeführtes Druckgas durchmischt. Durch diese Durchmischung wird die Fermentationsrate drastisch gesteigert. Im Sinne der vorliegenden Erfindung bedeutet "von oben in das Reaktionsmedium zuführen", dass Druckgas mittels geeigneter Vorrichtungen (wie mit Düsen versehenen Leitungsrohren), die von oben in das Reaktionsmedium hineinreichen, in das Reaktionsmedium geleitet werden. Die Länge der Leitungsrohre kann dabei so gewählt werden, dass sie im eingeführten Arbeitszustand (d. h. mit oder ohne eingeführtes Druckgas) in beliebiger Höhe unter die Oberfläche des Reaktionsmediums reichen.

Die Erfindung wird nachfolgend anhand der Figuren und Ausführungsbeispiele näher beschrieben. Dabei zeigt
- Fig. 1: einen Bioreaktor mit einem Druckbehälter für bioreaktive Stoffe und für Druckluft,
- Fig. 2: eine Stirnansicht des Bioreaktors,
- Fig. 3: eine Seitenansicht des Bioreaktors,
- Fig. 4: einen Schnitt durch den Fermentationsbehälter des Bioreaktors,
- Fig. 5: einen Schnitt entlang der Linie V-V in Fig. 4,
- Fig. 6: eine Medienmisch- und Druckluftzuleitungsanordnung für den Bioreaktor,
- Fig.: 7 eine schematische Ansicht einer mit dem Fermentationsbehälter verbundenen Messeinrichtung, und
- Fig. 8: den Reaktionsverlauf des in Beispiel 1 beschriebenen Fermentationsansatzes.

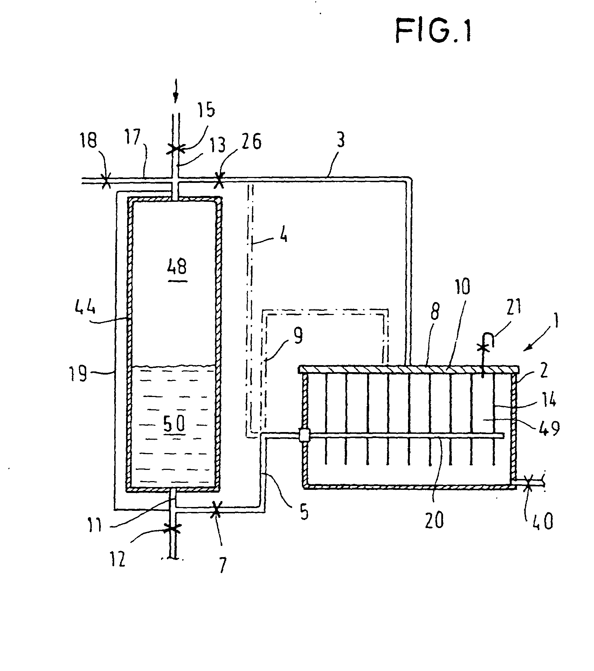

Der in Fig. 1 gezeigte Bioreaktor zur Fermentierung fester Stoffe und zur Durchführung eines nachfolgend näher beschriebenen Fermentationsverfahrens weist einen Fermentationsbehälter 2 auf, der mit einem Druckdeckel 8 druckdicht verschließbar ist.

Der Fermentationsbehälter 2 ist mit mindestens einer in den Reaktorraum 49 reichenden Düsenanordnung 10, 20 versehen, die über einen Druckbehälter 44, über eine Druckgasleitung 3 für eine vertikale Düsenanordnung 10 bzw. über eine Druckgasleitung 4 für eine horizontale Düsenanordnung 20 mit Druckluft 48 versorgt wird.

Der Druckbehälter 44 enthält zusätzlich einen bioreaktiven flüssigen Stoff 50, der über ein Absperrventil 7 und eine Druckleitung 5 der horizontalen Düsenanordnung 20 und/oder eine Druckleitung 9 der vertikalen Düsenanordnung 10 zugeführt werden kann.

Die vertikale Düsenanordnung 10 und/oder die horizontale Düsenanordnung 20 kann demzufolge auch als Zugabeeinrichtung für bioreaktive Stoffe alternativ zur Druckgasbeaufschlagung verwendet werden.

Am Boden des Druckbehälters 44 befindet sich eine Ablaufleitung 11 mit einem Absperrventil 12. Am oberen Ende des Druckbehälters 44 ist eine Zuführleitung 13 für die Zufuhr bioreaktiver Stoffe 50 sowie ein Absperrventil 15 angeordnet.

Das Druckgas 48, z.B. Druckluft, wird über eine Zuführleitung 17 und ein Absperrventil 18 zugeführt.

Seitlich neben dem Druckbehälter 44 ist ein Steigrohr 19 angeordnet.

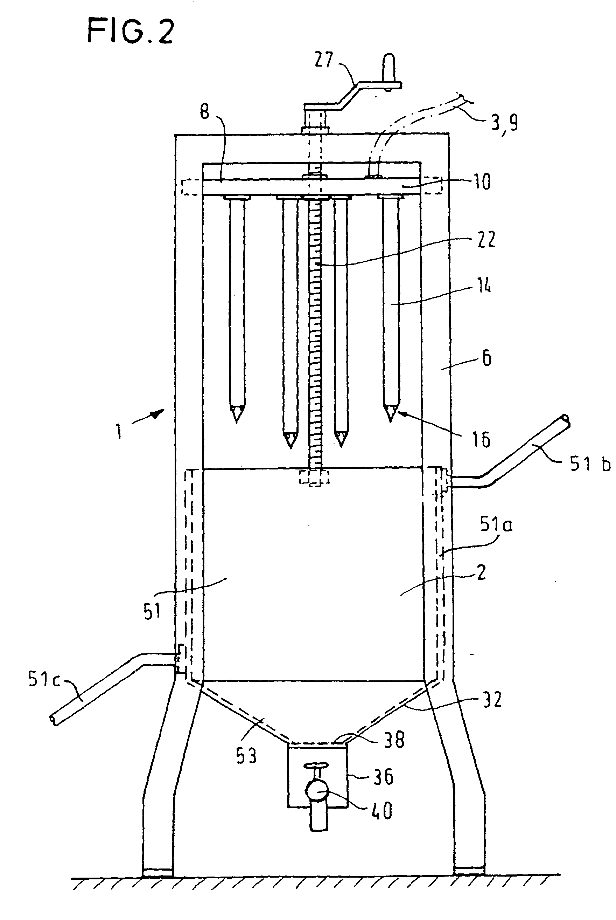

Der Fermentationsbehälter 2 weist des weiteren eine Druckausgleichseinrichtung 21 mit einem Absperrventil auf. Am Boden des Fermentationsbehälters 2 ist unterhalb eines konischen Bodenabschnitts 32 (Fig. 2) eine Ablaufrinne 36 vorgesehen, die mit einem Maschennetz 38 abgedeckt ist, so dass in dem Fermentationsbehälter 2 enthaltene Flüssigkeit vor ihrem Ablauf in die Ablaufrinne 36 grob gefiltert wird. Der Boden der Ablaufrinne 36 verläuft schräg zur Horizontalen, wobei an der Stirnseite 51 des Fermentationsbehälters an der tiefsten Stelle der Ablaufrinne 36 ein Ablaufventil 40 vorgesehen ist.

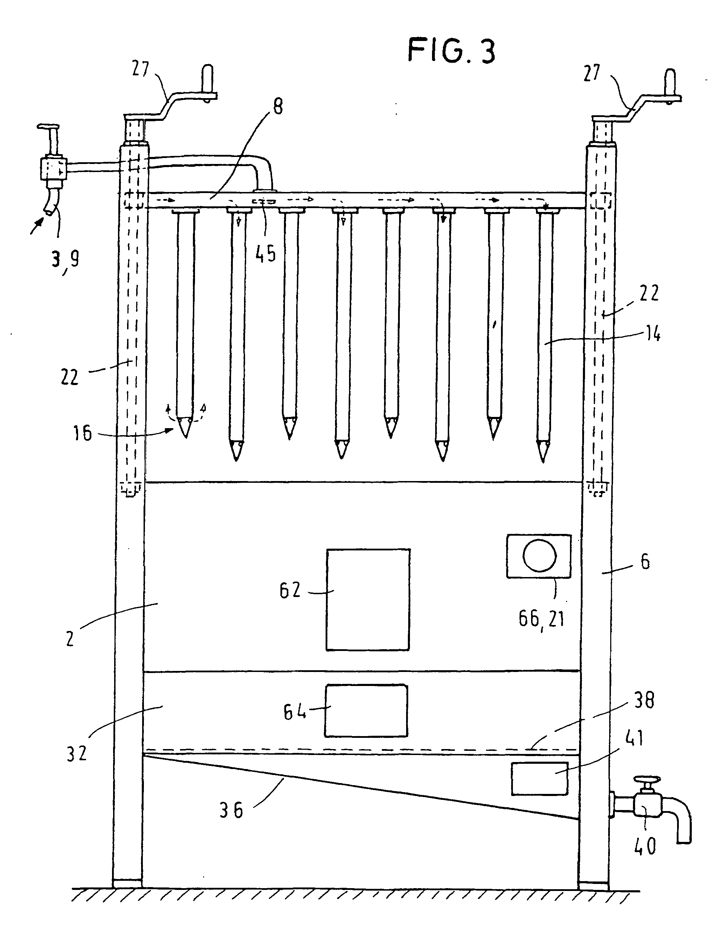

An der Stirnseite 51 des Fermentationsbehälters 2 befindet sich eine abnehmbare Klappe 53, um den Innenraum und das Maschennetz 38 reinigen zu können, ohne die den Reaktorraum 49 abschließende Deckplatte 8 öffnen zu müssen. An der Ablaufrinne 36 sind gegenüberliegend zwei Fenster 41 angebracht, die es ermöglichen mit einem optischen Sensor die optische Dichte des fermentierten Stoffes zu bestimmen (Fig. 3). Zusätzlich an den Seitenwänden angebrachte Fenster 62,64 ermöglichen eine visuelle Kontrolle der Fermentationsbedingungen, sie sind jedoch nicht obligatorisch.

Anstelle von oder zusätzlich zu einer nachfolgend beschriebenen externen Messkammer 72 kann an einer Seite des Fermentationsbehälters 2 eine abdichtbare Einrichtung 66 für regeltechnische Elektroden (z. B. pH, O₂-Partialdruck, Leitfähigkeit, Ionenspezifizität, usw.) sowie eine Druckausgleichseinrichtung 21 für den kontrollierten Ablass von Luft oder während der Fermentation entstehender Gase aus dem Fermentationsbehälter 2 angebracht sein.

Alle mit Medium oder Gasen in Kontakt kommenden Komponenten sind unabhängig voneinander autoklavierbar.

Der erfindungsgemäße Bioreaktor kann weiterhin Vorrichtungen zum Wärmeaustausch, d. h. Heizen und Kühlen aufweisen. Hierzu kann der Fermentationsbehälter 2 doppelwandig sein, so dass ein Hohlraum 51a ausgebildet ist. Dieser ist zwischen der inneren und äußeren Wand des Behälters 2 ausgebildet. Der Hohlraum 51a ist über ein Anschlussrohr 51b und Abflussrohr 51c, beispielsweise mit einem entsprechend temperierten Wärmetauschflüssigkeitsreservoir (z. B. Wasser- oder Ölreservoir) verbunden. Das Wasser oder Öl das durch einen externen Thermostaten eine definierte Temperatur aufweist, umströmt den Behälter 2. Durch diese Vorrichtung kann der Bioreaktor in einem Temperaturbereich von 10 bis 90°C stufenlos betrieben werden, wodurch zum Einen die für verschiedene Mikroorganismen optimale Temperatur im Bioreaktor eingestellt, und zum anderen der gesamte Bioreaktor samt Inhalt schonend sterilisiert bzw. tyndalisiert werden kann. Eine solche Sterilisierung/Tyndalisierung eines gesamten Bioreaktors in einem integrierten System, das als solches noch nicht beschrieben wurde, birgt deshalb deutlich Vorteile bei Anwendung des Systems.

Wie aus den Fig. 2 und 3 ersichtlich, wird der Fermentationsbehälter 2 von einem Gestell 6 gehalten. Das Gestell 6 nimmt auch zwei Spindeln 22 auf, mit deren Hilfe der Druckdeckel 8 des Fermentationsbehälters 2 auf diesen herabgelassen oder angehoben werden kann. Der Druckdeckel 8 bildet zugleich die vertikale Düsenanordnung, indem über den Druckdeckel 8 die Zufuhr von Druckgas oder bioreaktiven Stoffen zu den vertikal von dem Druckdeckel 8 abstehenden vertikalen Leitungsrohren 14 erfolgt. Die vertikalen Leitungsrohre 14 können unterschiedlich lang sein, wobei die Spitzen der Leitungsrohre 14 mit den Düsen 16 sich bei geschlossenem Druckdeckel 8 im Bereich des Boden des Fermentationsbehälters befinden. Durch das Absenken des Druckdeckels können die Düsen 16 auch feste Substrate im Reaktorraum penetrieren. In der geschlossenen Stellung des Druckdeckels kann der Fermentationsbehälter 2 druckdicht abgeschlossen werden.

Die Spindeln 22 werden mit Hilfe von an der Oberseite des Gestells 6 angeordneten Handkurbeln 27 betätigt.

Eine hohle Druckplatte 8 dient als Druckdeckel des Bioreaktors 1 und ist eine von zwei Möglichkeiten, den Bioreaktorraum im Fermentationsbehälter 2 zu belüften oder mit Medien zu versorgen. Sie kann vertikal von Schienen geführt sein und verschließt den Fermentationsbehälter 2 bei Betrieb hermetisch. Wenn der Bioreaktor 1 mit dem umzusetzenden Substrat beschickt werden soll, kann der Druckdeckel 8 neben der vorstehend beschriebenen mechanischen Art auch hydraulisch oder pneumatisch nach oben gefahren werden.

An der Oberseite hat der Druckdeckel 8 einen manuell oder elektronisch steuerbaren Anschluss für die Zufuhr von Druckluft (o. definierten Gasen). Optional kann der Anschluss auch für die Zufuhr von flüssigen definierten Medien, z. B. benötigte Co-Substrate, Vitamine, Mineralstoffe, Puffer oder Antibiotika genutzt werden. Die Luft trifft in dem Druckdeckel 8 zunächst auf eine Prallplatte 45. An der Unterseite des Druckdeckels 8 sind auswechselbare Leitungsrohre 14 mit Düsen 16 in Gewinde eingeschraubt. Baut sich ein Druck in dem Druckdeckel 8 auf, strömt die Luft kontinuierlich durch an den Spitzen der Leitungsrohre 14 befindliche Düsen 16 in den Reaktorraum 49 des Fermentationsbehälters 2 und belüftet diesen gleichmäßig. Neben der milden Durchmischung des Reaktorraums durch den kontinuierlichen Luftstrom, kann optional durch in definierten Intervallen gegebene starke Druckluftpulse eine deutliche Durchmischung des Reaktorraums erreicht werden.

Durch die unterschiedliche Länge der auswechselbaren, in den Druckdeckel 8 einschraubbaren Leitungsrohre 14 ist es möglich, den Reaktorraum 49 gezielt zu belüften. Durch bis zum Boden des Fermentationsbehälters 2 reichende Düsen 16 kann ein homogener, aerober Reaktionsraum geschaffen werden. Bei der Wahl kürzerer Düsen wird ein definierter Raum mit geringem Sauerstoff-Partialdruck erzeugt. Der Bioreaktor 1 kann entweder ohne Begasung (in diesem Fall können die Gewinde des Druckdeckels 8 durch Blindstopfen geschlossen werden) oder durch Zufuhr von definierten O₂-freien Gasen auch obligat anaerob gefahren werden. In diesem Fall sollten die möglicherweise zuzugebenden Medien vor Zugabe entgast werden.

Optional können flüssige Medien durch diese Düsen 16 auch aus dem Druck- oder Mischbehälter 44,46 in den Bioreaktor 1 eingelassen werden. Falls erforderlich, kann die Belüftung in diesem Fall durch die horizontal den Reaktorraum 49 durchziehende Düsenanordnung 20 erfolgen.

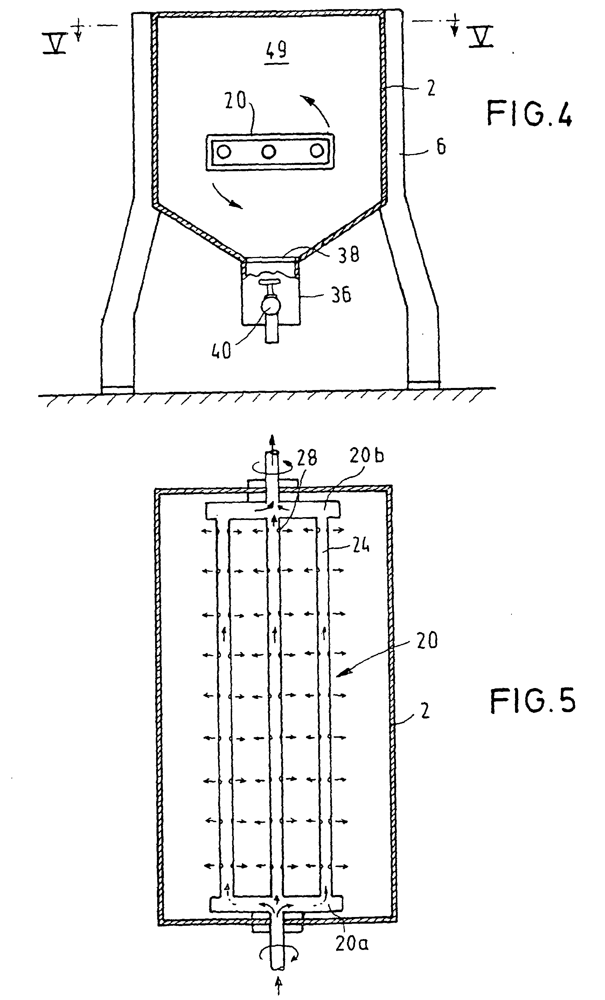

Die Fig. 4 und 5 zeigen die in dem Fermentationsbehälter 2 angeordnete horizontale Düsenanordnung 20, die ebenfalls entweder mit Druckluft 48 oder mit einem flüssigen bioreaktiven Stoff 50 beaufschlagt werden kann. Die horizontale Düsenanordnung 20 kann, wie aus Fig. 5 ersichtlich, einseitig in einer Stirnwand des Fermentationsbehälters 2 oder beidseitig in den Stirnwänden gelagert sein. Des weiteren kann vorgesehen sein, dass die horizontale Düsenanordnung in unterschiedlichen Höhen an der Stirnwand gelagert wird.

Wie aus Fig. 5 ersichtlich, besteht die horizontale Düsenanordnung 20 aus drei sich horizontal und parallel zueinander durch den Fermentationsbehälter 2 erstreckenden Leitungsrohren 24, die jeweils eine Vielzahl von Düsenöffnungen 28 aufweisen. Die drei Leitungsrohre 24 sind untereinander über eine Einlassverzweigung 20a und eine Auslassverzweigung 20b verbunden.

Wie aus Fig. 5 ersichtlich, kann die horizontale Düsenanordnung 20 um eine horizontale Drehachse gedreht werden, um zusätzlich eine gute Durchmischung des Behälterinhaltes zu erreichen. Bei Rotation der horizontalen Düsenanordnung 20 ist die Länge der Leitungsrohre 14 der vertikalen Düsenanordnung 10 so zu wählen, dass keine Kollision zwischen den Leitungsrohren 14 und den Leitungsrohren 24 auftreten kann. Alternativ kann - wenn keine Sterilität des Fermentationsvorgangs erforderlich ist - die vertikale Düsenanordnung so weit vertikal nach oben herausgefahren werden, dass keine Kollision zwischen den Leitungsrohren 14 und 24 auftreten kann. Für Einhaltung steriler Bedingungen ist die rotierbare horizontale Düsenanordnung 20 vorzugsweise an der tiefstmöglichen Stelle im Fermentationsbehälter 2 angeordnet. Die Leitungsrohre 14 sind dann vorzugsweise derart verkürzt, dass sie gerade nicht in den Drehbereich hineinragen. Die äußeren, benachbart zu den Seitenrändern des Fermentationsbehälters 2 angeordneten Leitungsrohre 14 sind hierbei länger als weiter innen angeordnete Leitungsrohre.

Die Auslassverzeigung 20b kann ein Ventil aufweisen, um den Auslass zu verschließen bzw. auch als Einlassverzweigung eingesetzt werden, wobei das in die Leitungsrohre 24 zugeführte Material vollständig durch die Düsenöffnungen 28 treten muss. Die Auslassverzweigung 20b kann auch vollständig entfallen. Dies erfordert allerdings einen stabileren Aufbau der Düsenanordnung 20.

Die Düsenanordnung 20 kann auch zum Heizen/Kühlen verwendet werden, indem die Düsen 28 verschlossen werden, oder die Düsenanordnung 20 durch ein entsprechendes Rohrsystem ohne Düsen ersetzt wird. Dabei dient die Einlassverzweigung 20a als Zufuhr der temperierten Heiz/Kühlflüssigkeit und die Auslassverzweigung 20b als Abfluss derselben. Eine derartige als Kühlanordnung ausgebildete Düsenanordnung 20 kann ebenfalls drehbar sein. Ferner können mehrere Düsen-/Kühlanordnungen vorgesehen sein, sodass gleichzeitig beispielsweise eine drehbare horizontale Düsenanordnung und eine drehbare horizontale Kühlanordnung in einem Behälter 2 angeordnet sein können.

Selbstverständlich ist es möglich, die vertikale Düsenanordnung 10 mit Druckgas 48 und die horizontale Düsenanordnung 20 mit dem bioreaktiven Stoff 50 und umgekehrt zu beaufschlagen.

Die zweite Düsenanordnung 20 durchzieht den Reaktorraum 49 horizontal. Sie kann in unterschiedlichen Höhen eingesetzt werden und ist um ihre Achse drehbar gelagert. Diese Düsenanordnung wird zur Versorgung des Bioreaktors mit flüssigen definierten Medien, z. B. Wasser, benötigte Co-substrate, Vitamine, Mineralstoffe, Puffer oder Antibiotika genutzt, kann aber optional zur Belüftung und Durchmischung des Bioreaktors 1 eingesetzt werden. Bei der Fermentation von flüssigen oder wasserlöslichen Substanzen kann die horizontale Düsenanordnung durch einen an der Außenseite des Bioreaktors 1 befindlichen Motor rotiert werden und so zu einer deutlichen Durchmischung des Bioreaktorinhalts beitragen. In diesem Fall werden kürzere vertikale Leitungsrohre 14 eingesetzt und die . horizontale Düsenanordnung 20 in deren unterster Arretierung eingesetzt.

Die Durchmischung des Bioreaktorinhalts bei der Fermentation fester Substanzen und die Versorgung des Bioreaktors 1 sowohl mit Luftsauerstoff oder definierten Gasen als auch mit Medium wird pneumatisch durch Druckluft erreicht. Über den Weg eines Druckbehälters 44, der in diesem Fall auch als Medienvorratstank verwendet wird, gelangt die Luft in den Druckdeckel 8 und von da aus in die vertikalen bzw. optional in die horizontalen Düsen 16,28. Zwischen dem Druckbehälter 44 und dem Druckdeckel 8 befindet sich ein Absperrventil 26. Wird das Absperrventil 26 geschlossen und der Absperrventil 7 am Boden des Druckbehälters 44 geöffnet, wird Medium durch die Druckluft über das horizontale und optional auch über die vertikale Düsenanordnung 10,20 in den Bioreaktor 1 gedrückt.

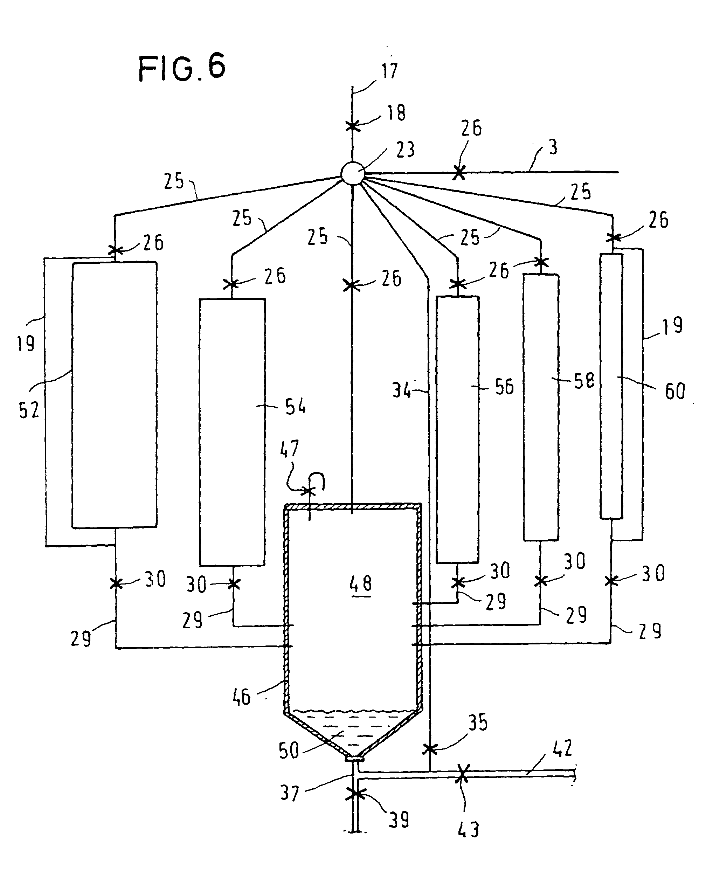

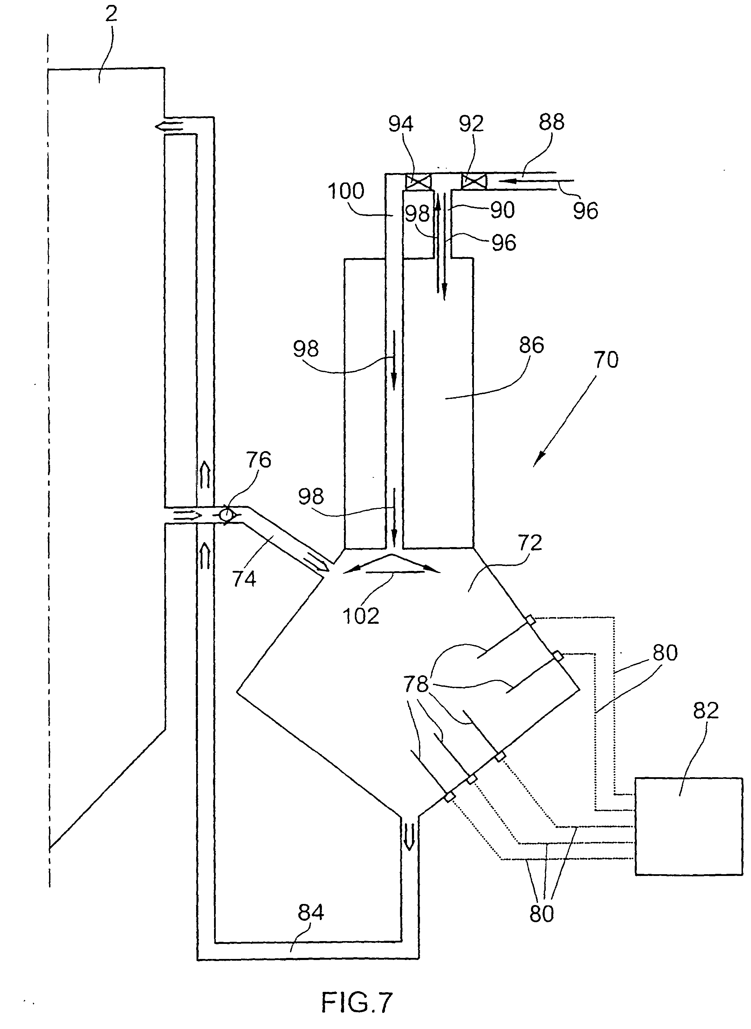

Fig. 6 zeigt ein Ausführungsbeispiel bei dem anstelle des Druckbehälters 44 ein Mischbehälter 46 an den Bioreaktor 1 angeschlossen ist. Mit dem Mischbehälter 46 sind mehrere Druckbehälter 52,54,56,58,60 verbunden, die unterschiedliche flüssige bioreaktive Stoffe, sowie Druckgas, z.B. Druckluft, enthalten. Diese unterschiedlichen Stoffe können dem Mischbehälter 46 zugeführt werden und in diesem in einem gewünschten Verhältnis gemischt werden. Über die Druckluftzuleitung 17 und das Absperrventil 18 gelangt Druckluft zu einer Verteileinrichtung 23, die die Druckluft 48 auf alle angeschlossenen Druckbehälter 52,54,56,58 und 60 sowie auf den Mischbehälter 46 verteilt. Die Druckluftzuleitungen 25 für die einzelnen Druckbehälter weisen jeweils ein Absperrventil 26 auf. Von der Verteileinrichtung 23 zweigt auch die Druckluftleitung 3 für den Fermentationsbehälter 2 ab.

Um unterschiedliche Medien gezielt in den vorgestellten Bioreaktor 1 einbringen zu können, wird das pneumatisches System aus mehreren Druckbehältern 52,54,56,58,60 unterschiedlichen Volumens genutzt. Fig. 6 zeigt eine Anwendung mit fünf austauschbaren und getrennt voneinander autoklavierbaren Druckbehältern (z. B. für Medium, zwei Puffer, Spurenelementlösung und Antibiotikalösung), bei denen graduierte Steigrohre 19 die jeweiligen Füllstände der Druckbehälter 52,54,56,58,60 anzeigen. Die Druckluft gelangt über eine Verteileinrichtung 23 in das pneumatische System. Ein Teil kann bei Bedarf zur Belüftung des Bioreaktors 1 direkt in die vertikale und/oder horizontale Düsenanordnung 10,20 geleitet werden. Die unabhängig voneinander regulierbaren Druckbehälter sind mit der Verteileinrichtung 23 verbunden. Die jeweiligen Medien aus den einzelnen Druckbehältern werden in einen Mischbehälter 46 gegeben. Ist der Mischbehälter mit den unterschiedlichen Medien gefüllt, wird der Druckausgleich über eine Druckausgleicheinrichtung 47 ermöglicht und durch eine weitere Druckgasleitung 34 der Verteileinrichtung 23 z.B. Druckluft in den Druckbehälterboden gepresst. Dadurch erfolgt die Durchmischung der unterschiedlichen Lösungen. Ist der Mischvorgang beendet, wird der Mischbehälter 46 unter Druckluft gesetzt und das gewünschte Medium in die horizontale und/oder vertikale Düsenanordnung 10,20 eingeleitet.

Jeder Druckbehälter kann mit einem Steigrohr 19 versehen sein. Die Zuführleitungen 29 für die bioreaktiven Stoffe aus den Druckbehältern weisen jeweils ein Absperrventil 30 auf.

Von der Verteileinrichtung 23 zweigt desweiteren eine Druckgasleitung 34 ab, die ein Absperrventil 35 enthält, wobei die Druckgasleitung 34 zu einer Abflussleitung 37 am Boden des Mischbehälters 46 führt, um für den Mischvorgang beispielsweise Druckluft zuzuführen. Die Abflussleitung ist mit einem Absperrventil 39 versehen. Von der Abflussleitung 37 zweigt eine Zuführleitung 42 mit einem Absperrventil 43 ab, über die die gemischten bioreaktiven Stoffe 50 dem Bioreaktor 1 zuführbar sind.

Der Mischbehälter 46 ist desweiteren mit einer Druckausgleicheinrichtung 47 für den Druckausgleich beim Mischvorgang versehen.

Das Druckgas kann der vertikalen und/oder horizontalen Düsenanordnung 10,20 auch in pulsierender Form zugeführt werden.

Mit dem Fermentationsbehälter 2 ist eine Messeinrichtung 70 verbunden. Die Messeinrichtung 70 weist eine Messkammer 72 auf, die über eine Zuführleitung 74 mit dem Fermentationsbehälter 2 verbunden ist. Durch die Zuführleitung 74 fließt aus dem Fermentationsbehälter 2 das zu messende Medium in die Messkammer 72. Um unabhängig von den Bedingungen in der Messkammer 72 die Fließrichtung aus dem Fermentationsbehälter 2 in die Messkammer 72 festzulegen, ist in der Zuführleitung 74 ein Rückschlagventil 76 angeordnet.

In der Messkammer 72 sind mehrere Messelektroden 78 vorgesehen, die über Leitungen 80 mit einer Messeinrichtung 82, die vorzugsweise computergesteuert ist, verbunden sind. Durch die Messelektroden 78 kann beispielsweise eine pH-, Sauerstoff-, Temperatur- und Ionen-Messung erfolgen.

Am Tiefpunkt der im Wesentlichen rautenförmigen Messkammer 72 ist mit der Messkammer 72 eine Rückführleitung 84 verbunden. Über die Rückführleitung 84 wird das gemessene Medium in den Fermentationsbehälter 2 zurückgeführt.

Zum Zurückführen des Mediums in den Fermentationsbehälter 2 wird die Messkammer 72 mit Druck beaufschlagt. Hierzu ist der Messkammer 72 eine Druckkammer 86 zugeordnet. Die Druckkammer 86 ist über Leitungen 88, 90 mit einer Druckluftquelle verbunden. Zur Erzeugung eines Überdrucks in der Druckkammer 86 ist ein Ventil 92 geöffnet und ein Ventil 94 geschlossen, so dass die Druckluft in Richtung der Pfeile 96 in die Druckkammer 86 strömt.

Um Medium aus der Messkammer 72 durch die Rückführleitung 84 in den Fermentationsbehälter 2 zurückzuführen, wird das Ventil 92 geschlossen und das Ventil 94 geöffnet. Hierdurch strömt die Druckluft aus der Druckkammer 86 in Richtung der Pfeile 98 durch eine mit der Messkammer 72 verbundenen Leitung 100 in die Messkammer 72 und erzeugt hierin einen Überdruck. Der definierte Überdruck in Druckkammer 86 ist zu dem Volumen der zu verdrängenden Flüssigkeit in der Messkammer 72 direkt proportional. Aufgrund des in der Messkammer 72 entstehenden Überdrucks wird das Rückschlagventil 76 in der Zuführleitung 74 geschlossen und das in der Messkammer 72 befindliche Medium wird durch die Rückführleitung 84 in den Fermentationsbehälter 2 zurückgedrückt. Um eine gleichmäßige Verteilung des Luftdrucks in der Messkammer zu erreichen und Verwirbellungen zu vermeiden, ist im Bereich des Einlasses der Druckluft in die Messkammer 72 ein Prallblech 102 vorgesehen.

Sobald das Medium aus der Messkammer 72 in den Fermentationsbehälter 2 zurückgefördert ist, wird das Ventil 94 wieder geschlossen und das Ventil 92 geöffnet. Hierdurch baut sich erneut ein Überdruck in der Druckkammer 86 auf. Sobald das Ventil 94 geschlossen ist, findet in der Messkammer 72 ein Druckausgleich statt, so dass das Rückschlagventil 76 durch aus dem Fermentationsbehälter 2 strömende Medium wieder geöffnet wird. Bei dem Ventil 76 kann es sich ferner um eine schaltbares Ventil handeln, um beispielsweise die der Messkammer 72 zugeführte Menge an zu messendem Medium zu steuern.

Die vorliegende Erfindung betrifft ebenfalls ein Verfahren zur aeroben Fermentierung fester Stoffe, wobei das diese festen Stoffe enthaltende Reaktionsmedium durch von oben in das Reaktionsmedium zugeführtes Druckgas 48 durchmischt wird. Dies erfolgt erfindungsgemäß dadurch, dass das Druckgas mittels geeigneter Vorrichtungen (wie z. B. eine vertikale Düsenanordnung 10, wie vorstehend beschrieben) direkt in das Reaktionsmedium eingeleitet wird, sodass die sich ausbreitenden Gasblasen eine Durchmischung bewirken. Da bei der aeroben Fermentation vorzugsweise sauerstoffreiches Druckgas (wie Luft, mit O₂ angereicherte Luft oder O₂) verwendet wird, wird durch dieses Verfahren auch der Sauerstoffgehalt in dem Reaktionsmedium erhöht, was üblicherweise die Fermentation zusätzlich beschleunigt. Das Einführen des Druckgases kann kontinuierlich (nachfolgend auch als "Belüftung" bezeichnet) oder durch Druckgaspulse (nachfolgend auch "Durchmischung bezeichnet) erfolgen.

"Feste Stoffe" im Sinne des erfindungsgemäßen Verfahrens sind dabei vorzugsweise Kohle, Holz und belastete Böden. Das erfindungsgemäße Verfahren ist insbesondere zur Fermentierung von Kohle, insbesondere von Braunkohle, geeignet. Die letztere besteht aus drei durch ihre unterschiedliche Löslichkeit in Abhängigkeit vom pH-Wert definierte Faktoren:
1. Huminsäuren, die durch 0,1 N NaOH-Lösung extrahierbar sind;
2. Fulvinsäuren, die auch im sauren Milieu löslich sind;
3. der als Matrix bezeichnete der unlösliche Rückstand.

Zur Verflüssigung der Braunkohle wird das Ausgangsprodukt, das gegebenenfalls vorbehandelt oder voroxidiert sein kann, in zermahlenem Zustand (Partikelgröße vorzugsweise 0,1 mm bis 2 cm, besonders bevorzugt 1 bis 10 mm) mit einer zur Solubilisierung ausreichenden Menge Lösungsmittel (d. h. Wasser oder Lösungsmittelsysteme auf wässriger Basis), Nährstoffe, Puffer (einschließlich Puffersubstanzen, Säuren, Basen) und Mikroorganismenkultur versetzt und unter Durchmischung mit sauerstoffhaltigem Druckgas kultiviert.

Geeignete Mikroorganismen für die Solubilisierung von Braunkohle sind dabei Schimmelpilze, Weißfäulepilze und Hefen. Ein bevorzugter Mikroorganismus für diesen Anwendungszweck ist *Trichoderma atroviride*. Die Nährstoffe die für dieses Fermentationsverfahren eingesetzt werden richten sich stark nach der Art des verwendeten Mikroorganismus. Bevorzugt ist dabei insbesondere die Zugabe von Kohlenstoffquellen zu Beginn der Reaktion an den die Braunkohle verflüssigenden Mikroorganismus, um einen Wachstumsvorteil zu gewährleisten.

Es werden Puffersubstanzen eingesetzt, die den gewünschten pH-Wert zum jeweiligen Reaktionszeitpunkt gewährleisten. So wird zu Beginn der Reaktion , wo ein pH von 5,5 bis 6,0 bevorzugt ist, und in der Verflüssigungsphase, wo ein pH von 6,5 bis 7,2 bevorzugt ist, vorzugsweise ein Citrat-/Phosphatpuffer mit einem pH von 3 verwendet, da der Pilz beim Wachstum das Medium selbst alkalisiert und nur gegentitriert werden muss. Besonders bevorzugt wird zu Versuchsbeginn ein pH von 5,5 eingestellt. Die Fermentation erfolgt bevorzugt bei einer Temperatur von 15 bis 35 °C.

In einer bevorzugten Ausführungsform wird die Braunkohle, das Braunkohle/Lösungsmittelgemisch oder das Braunkohle/Lösungsmittel/Nährmittelgemisch vor der Zugabe der fermentierenden Mikroorganismen sterilisiert bzw. tyndalisiert. Dies erfolgt vorzugsweise durch mehrere Zyklen eines mindestens 45minütigen Erhitzens auf Temperaturen über 75 °C, vorzugsweise über 80 °C, und nachfolgende Abkühlung für mehrere Stunden auf Raumtemperatur.

Bei den 80°C Schritten wurden die physiologisch aktiven Mikroorganismen in Substrat, Medium und Reaktorraum, nicht jedoch ruhende Sporen abgetötet. In den Zeiten mit moderaten Temperaturen keimten die Sporen und wurden in dem dann folgenden anschließenden Hitzeschritt abgetötet.

Beim konventionellen Tyndalisieren wird ein dreimaliges Erhitzen einer Flüssigkeit oder eines Nährmediums verstanden. In den Intervallen zwischen den Temperaturschritten wird das Gut bei Raumtemperatur aufbewahrt (Eckhard Bast, 1999, Mikrobiologische Methoden: eine Einführung in grundlegende Arbeitstechniken - Heidelberg, Berlin; Spektrum, Akad. Verl. ISBN 3-8274-0786-9).

Die gemäß dem vorliegenden Verfahren erhältliche mikrobiell solubilisierte Kohle kann als Kohlenstoff- und Energiequelle für Bakterien verwendet werden, die in der Lage sind, aus dem chemisch heterogenen Massenprodukt Kohle einen chemisch charakterisierten Stoff wie z. B. Polyhydroxyfettssäuren für die Synthese biologisch abbaubarer Kunststoffe zu produzieren (A. Steinbüchel und B. Füchtenbusch, Proceedings ICCS 97, 1673 - 1676 (1997)). Der aliphatische, mikrobiell nicht zu verflüssigende Rest, der einen geringeren Wasser- und Ascheanteil und somit einen höheren spezifischen Brennwert aufweist, kann zum einen für die direkte thermische Nutzung (R. Köpsel et al., Freiberger Forschungshefte, 159 - 166 (1998)) zum anderen für weitere anschließende Fermentationsprozesse durch aliphatenabbauende Hefen genutzt werden (U. Hölker et al., Proceedings of the 16^{th} SMYTE, Slowakei, S. 16 (1998); Folia Microbiol 44, 226 - 227 ( 1999)).

Besonders bevorzugt wird das erfindungsgemäße Verfahren in dem vorstehend beschriebenen Bioreaktor durchgeführt.

Die vorliegende Erfindung wird anhand des nachfolgenden Beispiels näher erläutert.

### Beispiele

### Allgemeine Verfahren

Das Produkt "verflüssigte Kohle" wurde als der Probeüberstand definiert, der nach 20 min Zentrifugieren bei 10 000 g erhalten wurde. Der Grad der Verflüssigung wurde mittels der optischen Dichte bei 450 nm oder über das Trockengewicht des Überstands bestimmt. Um die Huminsäureverbindungen von Fulvinsäureverbindungen zu trennen, wurde der Überstand auf pH 1,5 angesäuert und erneut zentrifugiert. Die verflüssigten Produkte wurden bezüglich ihrer optischen Dichte, Huminsäure- und Fulvinsäuregehalt und der bakteriellen Verunreinigung mittels Inkubation von Proben in Vollmedien und anschließender mikroskopischer Analyse charakterisiert.

### Beispiel 1: Fermentation von Braunkohle

Ein erfindungsgemäßer Bioreaktor, wie in Fig. 1 bis 6 gezeigt, mit einer vertikalen Düsenordnung (10), einem Druckdeckel (8) mit bis zum Boden des Reaktorraums (49) reichenden Leitungsrohren (14) mit Düsen (16) und einer starren horizontalen Düsenanordnung (20); Volumen 25 I) wurde mit 2500 g Braunkohle (Bergheim Lithotyp A, Partikelgröße 2 - 10 mm, Wassergehalt der Kohle ca. 50 %) als umzusetzender Feststoff in 10 I Wasser beschickt. Als kohlesolubilisierender aerober Pilz wurde *Trichoderma atroviride* eingesetzt (U. Hölker et al., Fuel Processing Technol., 52 65 - 71 (1997)). 50 g Glutamat wurden zugegeben, um die Induktion kohleverflüssigender Enzyme einzuleiten (U. Hölker et al., Appl. Microbiol. Biotechnol. 44, 351 - 255 (1995)). Der Anfangs-pH betrug 5,8, um dem Pilz gegenüber in der Kohle befindlichen Bakterien zunächst einen Wachstumsvorteil zu geben. Der zur Belüftung ausreichende kontinuierliche Luftdruck betrug 0,4 bar. Täglich wurde der Luftdruck durch die vertikale Düsenanordnung 10 für 10 s auf 3 bar erhöht, um den Reaktorinhalt zu durchmischen.

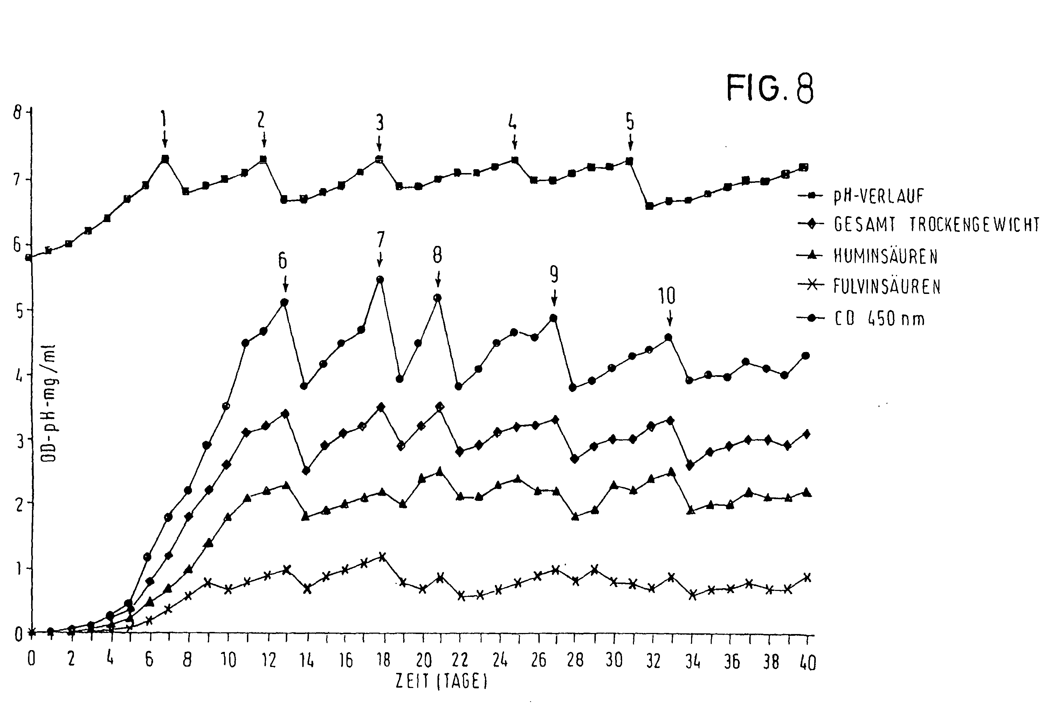

In einem semikontinuierlichen Ansatz wurden in Abständen von 24 Stunden über die horizontale Düsenanordnung (20) 800 ml Wasser zugegeben und eine gleiche Menge Reaktorinhalt am Ablaufsystem entnommen. In dieser Suspension wurde die optische Dichte, der pH-Wert, der Huminund Fulvinsäureanteil bestimmt und auf bakterielle Kontamination überprüft. Erreichte der pH-Wert 7,3 wurde er über das Medienzulaufsystem auf 7,0 zurücktitriert (Fig. 8, Pfeile 1 - 5).

Die in diesem Fermentationsansatz angestrebte Ausbeute an solubilisierter Kohle betrug 3 mg Trockengewicht pro ml Suspension bei pH 7 pro Tag und sollte kontinuierlich über einen Zeitraum von 30 Tagen hinweg konstant gehalten werden. Dies entspricht einer angestrebten Ausbeute von ca. 2 g Solubilisationsprodukte pro Tag. Bei einem Anstieg über 3,3 mg Trockenmasse pro ml wurde durch Wasserzugabe über das Medienzulaufsystem der Reaktorinhalt verdünnt und wieder auf die gewünschte Konzentration eingestellt (Fig. 8, Pfeile 6 - 10).

Nach einer Fermentationszeit von 12 Tagen wurde die angestrebte Konzentration solubilisierter Kohle erreicht und in einem Zeitraum von weiteren 28 Tagen wurden 71 g Solubilisierungsprodukte im vorgeschlagenen Bioreaktor produziert.

### Beispiel 2: Verfahren zur schonenden Sterilisierung von Bioreaktoren samt Inhalt; modifizierte Tyndalisierung

Ein erfindungsgemäßer Bioreaktor (wie in Fig. 1 bis 6 gezeigt, mit einer einführbaren vertikalen Düsenordnung (10) mit kurzen Leitungsrohren (14), Vorrichtungen zum Wärmeaustausch (51a, 51b, 51c) in der Außenwand einer rotierbaren horizontalen Düsenanordnung (20) und einer Messeinrichtung (70); Vol. 12,5 I) wurde, exakt wie in Beispiel 1 beschrieben, beschickt. Der Druckdeckel (8) wurde geschlossen und die Düsen (16) somit pneumatisch in das Substrat gepresst. Das pneumatische Umwälz- und Mess-System wurde aktiviert und im Minutenintervall wurde das Medium an dem Temperaturfühler der Messkammer vorbei und durch das horizontale Düsensystem (20) wieder in den Bioreaktor zurückgepumpt. Wasser wurde im Thermostaten auf 95°C erhitzt und durch den Mantel des Bioreaktors (51a, 51b, 51c) gepumpt, bis die Temperatur im Innenraum 80°C erreichte. Diese Temperatur wurde 45 min gehalten (unter ständiger Durchlüftung/Durchmischung durch das vertikale Düsensystem (10) und Umwälzens des Mediums durch das horizontale Düsensystem (20)). Anschließend wurde der Bioreaktor durch temperiertes Wasser in der Ummantelung auf 25°C gekühlt, dabei 12 Stunden belüftet, durchmisch und umgewälzt. Anschließend wurde die Temperatur im Bioreaktorraum wie zuvor erneut für 45 Minuten auf 80°C eingestellt. Es folgte eine erneutes Abkühlen für 20 Stunden auf 25°C, gefolgt von einem dritten Erhitzen des Reaktorinnenraums auf 80°C für 45min

Um den Sterilitätserfolg zu überprüfen, wurde der Bioreaktor anschießend weiter durchmischt, belüftet und umgewälzt. Täglich (7 Tage) wurden 1 ml-Proben entnommen und mit diesen sowohl Petrischalen (1,2% Agar) als auch 50ml-Flüssigkulturen, die das im Bioreaktor verwendete Nährmedium enthielten, angeimpft. Die Petrischalen wurden 72 Stunden bei 25°C, die Flüssigkulturen bei 25°C und 120 rpm auf dem Schüttler inkubiert. Es zeigte sich, dass keine Kontaminationen unter diesen Bedingungen im Reaktorraum detektierbar waren.

Nachdem die Sterilitätskontrolle durchgeführt war, wurde der Bioreaktor mit dem kohlesolubilisierenden Pilz *T. atroviride* angeimpft und die Kohle analog Beispiel 1, jedoch bei einer eingestellten Temperatur von 25°C fermentiert. Es wurde 60 g Verflüssigungsprodukt erhalten.

## Patentansprüche

1. Bioreaktor zur Fermentierung fester Substrate mit einem Fermentationsbehälter (2), einer Zugabeeinrichtung für bioreaktive Stoffe und einer Düsenanordnung in dem Fermentationsbehälter (2) zur Belüftung und Durchmischung der Substrate, wobei die mindestens eine Düsenanordnung (10,20) eine Vielzahl von parallel in den Reaktionsraum (49) des Fermentationsbehälters (2) hineinragende und mit Düsen (16,28) versehenen Leitungsrohren (14,24) aufweist, wobei eine erste, sich vertikal erstreckende Düsenanordnung (10) in den Reaktionsraum (49) des Fermentationsbehälters (2) ein- und ausfahrbar ist, **gekennzeichnet durch** eine zweite horizontale Düsenanordnung (20) aus mindestens zwei sich horizontal und zueinander parallel **durch** den Reaktionsraum (49) erstreckenden, miteinander verbundenen Leitungsrohren (24), die jeweils eine Vielzahl von Düsenöffnungen (28) aufweisen, vorgesehen ist.

2. Bioreaktor nach Anspruch 1, **dadurch gekennzeichnet, dass** die horizontale Düsenanordnung (20) um eine horizontale Drehachse drehbar ist.

3. Bioreaktor nach Anspruch 1 oder 2, **dadurch gekennzeichnet, dass** der Fermentationsbehälter (2) einen im Querschnitt sich verjüngenden Bodenabschnitt (32) aufweist.

4. Bioreaktor nach Anspruch 3, **dadurch gekennzeichnet, dass** der konisch gestaltete Bodenabschnitt (32) in einer zur Horizontalen schräg verlaufenden Ablaufrinne (36) mündet, die an der tiefsten Stelle ein Ablaufventil (40) aufweist.

5. Bioreaktor nach einem oder mehreren der Ansprüche 1 bis 4, **dadurch gekennzeichnet, dass** die mindestens eine Düsenanordnung (10,20) Druckgas (48) aus einem Druckbehälter (44,46) erhält.

6. Bioreaktor nach Anspruch 5, **dadurch gekennzeichnet, dass** in dem Druckbehälter (44,46) außer dem Druckgas (48) ein bioreaktiver flüssiger Stoff (50) enthalten ist.

7. Bioreaktor nach Anspruch 6, **dadurch gekennzeichnet, dass** die mindestens eine Düsenanordnung (10,20) alternativ Druckluft (48) oder den flüssigen bioreaktiven Stoff (50) aus dem Druckbehälter (44,46) erhält.

8. Bioreaktor nach einem oder mehreren der Ansprüche 1 bis 7, **dadurch gekennzeichnet, dass** die mindestens eine Düsenanordnung (10,20) mit pulsierender Druckluft (48) beaufschlagbar ist.

9. Bioreaktor nach einem oder mehreren der Ansprüche 1 bis 8, **dadurch gekennzeichnet, dass** die zweite Düsenanordnung (20) höhenverstellbar in dem Fermentationsbehälter (2) angeordnet ist.

10. Bioreaktor nach Anspruch 6, **dadurch gekennzeichnet, dass** mehrere mit Druckluft (48) beaufschlagte und mit einem Mischbehälter (46) verbundene Druckbehälter (52,54,56,58,60) vorgesehen sind, die unterschiedliche flüssige bioreaktive Stoffe enthalten.

11. Bioreaktor nach Anspruch 10, **dadurch gekennzeichnet, dass** der Mischbehälter (46) eine Druckausgleichseinrichtung (47) aufweist.

12. Bioreaktor nach Anspruch 10 oder 11, **dadurch gekennzeichnet, dass** die Druckbehälter (52,54,56,58,60) austauschbar und getrennt voneinander autoklavierbar sind.

13. Bioreaktor nach einem oder mehreren der Ansprüche 4 bis 12, **dadurch gekennzeichnet, dass** die Ablaufrinne (36) mit einem Maschennetz (38) abgedeckt ist.

14. Bioreaktor nach einem der oder mehreren der Ansprüche 1 bis 13, **dadurch gekennzeichnet, dass** ein Druckdeckel (8) des Fermentationsbehälters (2) die erste Düsenanordnung (10) aufnimmt, deren Leitungsrohre (24) vertikal von dem Druckdeckel (8) in den Reaktionsraum (49) hineinragen.

15. Bioreaktor nach Anspruch 14, **dadurch gekennzeichnet, dass** die vertikalen Leitungsrohre (14) der ersten Düsenanordnung (10) auswechselbar in dem Druckdeckel (8) angeordnet sind.

16. Bioreaktor nach einem oder mehreren der Ansprüche 1 bis 15, **dadurch gekennzeichnet, dass** der Fermentationsbehälter (2) über eine Zuführleitung (74) mit einer Messkammer (72) und diese über eine Rückführleitung (84) mit dem Fermentationsbehälter (2) verbunden ist, und dass die Messkammer (72) zum Zurückführen von gemessenem Medium mit Druck beaufschlagbar ist.

17. Bioreaktor nach einem oder mehreren der Ansprüche 1 bis 16, **dadurch gekennzeichnet, dass** eine Vorrichtung zum Wärmeaustausch vorgesehen ist, insbesondere mit einer Vorrichtung,
(i) in der der Fermentationsbehälter (2) doppelwandig ist und der so gebildete Hohlraum (51a) über ein Anschlussrohr (51b) und einem Abflussrohr (51c) mit temperierter Wärmetauschflüssigkeiten durchströmbar ist und/oder
(ii) die ein horizontales Rohrsystem (20) in den Fermentationsbehälter (2) ist, die mit temperierbarer Wärmeaustauschflüssigkeit durchströmbar ist.

18. Verfahren zur aeroben Fermentierung fester Stoffe, wobei in einem wie in den Ansprüchen 1 bis 17 definierten Bioreaktor ein die festen Stoffe enthaltendes Reaktionsmedium durch von oben in das Reaktionsmedium zugeführtes Druckgas (48) durchmischt wird.

19. Verfahren nach Anspruch 18, wobei das Durchmischen durch einen kontinuierlichen Druckgasstrom oder durch Druckgaspulse erfolgt.

20. Verfahren nach Anspruch 18 oder 19, wobei die festen Stoffe ausgewählt sind aus Kohle, Holz und belasteten Böden.

21. Verfahren nach Anspruch 20, wobei der feste Stoff Kohle und insbesondere Braunkohle ist.

22. Verfahren nach Anspruch 21, wobei dem Reaktionsgemisch weiterhin ein zur Fermentation geeigneter Mikroorganismus, Nährstoffe und/oder Puffer zugegeben werden.

23. Verfahren nach Anspruch 21, wobei die Braunkohle oder das Braunkohle enthaltende Reaktionsmedium samt Bioreaktor vor der Fermentation oder der Zugabe des Mikroorganismus tyndalisiert wird.

24. Verfahren nach einem oder mehreren der Ansprüche 21 bis 23, wobei
(i) die Braunkohle eine Partikelgrößen von 1 bis 10 mm aufweist;
(ii) der Mikroorganismus ausgewählt ist aus Schimmelpilzen, Hefen und Weißfäulepilzen und insbesondere *Trichoderma atroviride* ist;
(iii) der pH des Reaktionsmediums bei Reaktionsbeginn von 5.5 bis 6.0 beträgt;
(iv) der pH während der Solubilisierungsphase auf 6.5 bis 7.2 gehalten wird;
(v) die Fermentation bei einer Temperatur von 25 °C bis 30 °C erfolgt und/oder
(vi) 1 bis 25 I Druckluft pro I Fermentationslösung pro Tag durch das Reaktionsmedium geleitet werden.

## Claims

1. A bioreactor for fermenting solid substrates, comprising a fermentation vessel (2), a charging means for bioreactive substances, and a nozzle arrangement within said fermentation vessel (2) for aeration and thorough mixing of the substrates, wherein the at least one nozzle arrangement (10, 20) has a multitude of pipes (14, 24) extending in parallel into the reaction space (49) of the fermentation vessel (2) and provided with nozzles (16, 28), wherein a first, vertically extending nozzle arrangement (10) can be extended into and retracted from the reaction space (49) of the fermentation vessel (2), **characterized by** a second, horizontal nozzle arrangement (20) consisting of at least two interconnected pipes (24) each having a plurality of nozzle orifices (28) and extending horizontally and in parallel through the reaction space (49).

2. The bioreactor according to claim 1, **characterized in that** said horizontal nozzle arrangement (20) can be rotated around a horizontal rotation axis.

3. The bioreactor according to claim 1 or 2, **characterized in that** said fermentation vessel (2) has a bottom section (32) with a tapering cross-section.

4. The bioreactor according to claim 3, **characterized in that** said conically designed bottom section (32) leads into a draining channel (36) which is inclined from horizontal and has a draining valve (40) at the lowest position thereof.

5. The bioreactor according to one or more of claims 1 to 4, **characterized in that** said at least one nozzle arrangement (10, 20) receives compressed gas (48) from a pressure vessel (44, 46).

6. The bioreactor according to claim 5, **characterized in that** said pressure vessel (44, 46) contains a bioreactive liquid substance (50) in addition to said compressed gas (48).

7. The bioreactor according to claim 6, **characterized in that** said at least one nozzle arrangement (10, 20) alternatively receives compressed air (48) or said liquid bioreactive substance (50) from said pressure vessel (44, 46).

8. The bioreactor according to one or more of claims 1 to 7, **characterized in that** said at least one nozzle arrangement (10, 20) can be pressurized with pulsing compressed air (48).

9. The bioreactor according to one or more of claims 1 to 8, **characterized in that** said second nozzle arrangement (20) is provided within said fermentation vessel (2) in a height-adjustable manner.

10. The bioreactor according to claim 6, **characterized in that** a multitude of pressure vessels (52, 54, 56, 58, 60) pressurized with compressed air (48) and connected to a mixing vessel (46) are provided which contain different liquid bioreactive substances.

11. The bioreactor according to claim 10, **characterized in that** said mixing vessel (46) has a pressure compensating means (47).

12. The bioreactor according to claim 10 or 11, **characterized in that** said pressure vessels (52, 54, 56, 58, 60) are exchangeable and can be separately autoclaved.

13. The bioreactor according to one or more of claims 4 to 12, **characterized in that** said draining channel (36) is covered by a wire mesh (38).

14. The bioreactor according to one or more of claims 1 to 13, **characterized in that** a pressure lid (8) of said fermentation vessel (2) accommodates said first nozzle arrangement (10) whose pipes (24) extend vertically from the pressure lid (8) into the reaction space (49).

15. The bioreactor according to claim 14, **characterized in that** said vertical pipes (14) of said first nozzle arrangement (10) are provided in said pressure lid (8) to be exchangeable.

16. The bioreactor according to one or more of claims 1 to 15, **characterized in that** said fermentation vessel (2) is connected through a feed line (74) with a measuring chamber (72), which is again connected through a recirculating line (84) with said fermentation vessel (2), and that said measuring chamber (72) can be pressurized for recirculating measured media.

17. The bioreactor according to one or more of claims 1 to 16, **characterized in that** a device for heat exchange is provided, especially comprising a device
(i) in which said fermentation vessel (2) has a double wall and the thus formed cavity (51a) can be flowed through with temperature-controlled heat exchange fluids through a connecting pipe (51b) and discharge pipe (51c); and/or
(ii) which is a horizontal pipe system (20) within said fermentation vessel (2) which can be flowed through with a temperature-controlled heat exchange fluid.

18. A method for the aerobic fermentation of solid substrates, wherein a reaction medium containing such solid substrates is thoroughly mixed by compressed gas (48) supplied to the reaction medium from above in a bioreactor as defined in any of claims 1 to 17.

19. The method according to claim 18, wherein said thorough mixing is effected by a continuous stream of compressed gas or by compressed gas pulses.

20. The method according to claim 18 or 19, wherein said solid substances are selected from coal, wood and loaded soils.

21. The method according to claim 20, wherein said solid substance is coal, especially brown coal (lignite).

22. The method according to claim 21, wherein a microorganism suitable for fermentation, nutrients and/or buffers are further added to the reaction mixture.

23. The method according to claim 21, wherein said brown coal or the reaction medium containing said brown coal is tyndallized together with the bioreactor prior to fermentation or prior to the addition of said microorganism.

24. The method according to one or more of claims 21 to 23, wherein:
(i) said brown coal has a particle size of from 1 to 10 mm;
(ii) said microorganism is selected from molds, yeasts and white rot fungi, especially *Trichoderma atroviride*;
(iii) the pH of the reaction medium is from 5.5 to 6.0 at the beginning of the reaction;
(iv) the pH is maintained at from 6.5 to 7.2 during the solubilization phase;
(v) the fermentation is performed at a temperature of from 25 °C to 30 °C; and/or
(vi) from 1 to 25 I of compressed air per liter of fermentation broth per day is passed through the reaction medium.

## Revendications

1. Bioréacteur de fermentation de matières solides comportant une cuve de fermentation (2), un dispositif d'amenée des substances bioréactives et un agencement de buses dans la cuve de fermentation (2) pour la ventilation et le mélange des matières, dans lequel l'au moins un agencement de buses (10, 20) présente une pluralité de tubes de conduite (14, 24) faisant saillie parallèlement dans la chambre de réaction (49) de la cuve de fermentation (2) et munis de buses (16, 28), dont un premier agencement de buses (10) s'étendant verticalement pénètre dans la chambre de réaction (49) de la cuve de fermentation (2) et en sort, **caractérisé par** un deuxième agencement de buses (20) horizontal, constitué d'au moins deux tubes de conduite (24) reliés l'un à l'autre, s'étendant horizontalement et parallèlement l'un à l'autre à travers la chambre de réaction (49), qui présentent respectivement une pluralité d'ouvertures de buses (28).

2. Bioréacteur selon la revendication 1, **caractérisé en ce que** l'agencement de buses (20) horizontal est rotatif autour d'un axe de rotation horizontal.

3. Bioréacteur selon la revendication 1 ou 2, **caractérisé en ce que** la cuve de fermentation (2) présente un segment de fond (32) dont la section va en se rétrécissant.

4. Bioréacteur selon la revendication 3, **caractérisé en ce que** le segment de fond (32) conçu en forme conique débouche dans une rigole d'écoulement (36) s'étendant en oblique par rapport à l'horizontale, qui présente une vanne d'écoulement (40) à l'emplacement le plus bas.

5. Bioréacteur selon l'une ou plusieurs des revendications 1 à 4, **caractérisé en ce que** l'au moins un agencement de buses (10, 20) contient du gaz comprimé (48) provenant d'un réservoir sous pression (44, 46).

6. Bioréacteur selon la revendication 5, **caractérisé en ce qu'**une substance liquide bioréactive (50) est contenue dans le réservoir sous pression (44, 46) en plus du gaz comprimé (48).

7. Bioréacteur selon la revendication 6, **caractérisé en ce que** l'au moins un agencement de buses (10, 20) reçoit alternativement de l'air comprimé (48) ou la substance liquide bioréactive (50) provenant du réservoir sous pression (44, 46).

8. Bioréacteur selon l'une ou plusieurs des revendications 1 à 7, **caractérisé en ce que** l'au moins un agencement de buses (10, 20) peut être sollicité par de l'air comprimé (48) pulsé.

9. Bioréacteur selon l'une ou plusieurs des revendications 1 à 8, **caractérisé en ce que** le deuxième agencement de buses (20) est disposé de façon réglable en hauteur dans la cuve de fermentation (2).

10. Bioréacteur selon la revendication 6, **caractérisé en ce que** plusieurs réservoirs sous pression (52, 54, 56, 58, 60) sollicités par l'air comprimé (48) et reliés à un réservoir de mélange (46) sont prévus, qui contiennent différentes substances bioréactives liquides.

11. Bioréacteur selon la revendication 10, **caractérisé en ce que** le réservoir de mélange (46) présente un dispositif de compensation de pression (47).

12. Bioréacteur selon la revendication 10 ou 11, **caractérisé en ce que** les réservoirs sous pression (52, 54, 56, 58, 60) sont remplaçables et peuvent être autoclavés séparément les uns des autres.

13. Bioréacteur selon l'une ou plusieurs des revendications 4 à 12, **caractérisé en ce que** la rigole d'écoulement (36) est recouverte d'un réseau maillé (38).

14. Bioréacteur selon l'une ou plusieurs des revendications 1 à 13, **caractérisé en ce qu'**un couvercle sous pression (8) de la cuve de fermentation (2) loge le premier agencement de buses (10), dont les tubes de conduite (24) passent verticalement du couvercle sous pression (8) dans la chambre de réaction (49).

15. Bioréacteur selon la revendication 14, **caractérisé en ce que** les tubes de conduite (14) verticaux du premier agencement de buses (10) sont disposés de façon remplaçable dans le couvercle sous pression (8).

16. Bioréacteur selon l'une ou plusieurs des revendications 1 à 15, **caractérisé en ce que** la cuve de fermentation (2) est reliée, par une conduite d'amenée (74), à une chambre de mesure (72) et celle-ci est reliée par une conduite de retour (84) à la cuve de fermentation (2), et **en ce que** la chambre de mesure (72) est sollicitée par la pression pour le retour du fluide mesuré.

17. Bioréacteur selon l'une ou plusieurs des revendications 1 à 16, **caractérisé en ce qu'**il est prévu un dispositif pour l'échange thermique, en particulier avec un dispositif
(i) dans lequel la cuve de fermentation (2) est à double paroi et l'espace creux (51a) ainsi formé peut être traversé par des liquides tempérés échangeurs de chaleur, au moyen d'un tube de raccordement (51b) et d'un tube d'écoulement (51c) et/ou
(ii) qui est un système de tube horizontal (20) dans la cuve de fermentation (2), qui peut être traversé par un liquide tempéré échangeur de chaleur.

18. Procédé de fermentation aérobie de matières solides, dans lequel, dans un bioréacteur tel que défini dans les revendications 1 à 17, un milieu réactionnel contenant les matières solides, est mélangé par le gaz sous pression (48) amené du haut dans le milieu réactionnel.

19. Procédé selon la revendication 18, dans lequel le mélange est réalisé au moyen d'un écoulement continu de gaz comprimé ou d'impulsions de gaz comprimé.

20. Procédé selon la revendication 18 ou 19, dans lequel les matières solides sont choisies parmi le charbon, le bois et les sols contaminés.

21. Procédé selon la revendication 20, dans lequel la matière solide est du charbon et en particulier du lignite.

22. Procédé selon la revendication 21, dans lequel un microorganisme approprié pour la fermentation, des substances nutritives et/ou un tampon sont ajoutés également au mélange réactionnel.

23. Procédé selon la revendication 21, dans lequel le lignite ou le milieu réactionnel contenant le lignite et le bioréacteur sont tyndallisés avant la fermentation ou l'addition du microorganisme.

24. Procédé selon l'une ou plusieurs des revendications 21 à 23, dans lequel
(i) le lignite présente une granulométrie de 1 à 10 mm ;
(ii) le microorganisme est choisi parmi des moisissures, des levures et des champignons de pourriture alvéolaire et en particulier *Trichoderma atroviride* ;
(iii) le pH du milieu réactionnel est 5,5 à 6,0 au début de la réaction ;
(iv) le pH pendant la phase de solubilisation est maintenu à 6,5 à 7,2 ;
(v) la fermentation a lieu à une température de 25°C à 30°C et/ou
(vi) 1 à 25 litres d'air comprimé par litre de solution de fermentation et par jour sont amenés à travers le milieu réactionnel.
